# EUROPEAN PATENT SPECIFICATION

(11) **EP 4 041 210 B1**
(45) Date of publication and mention of the grant of the patent: **18.03.2026**
(21) Application number: 20877977.7
(22) Date of filing: 13.10.2020
(51) Int. Cl.: A61K 31/05, A61K 31/165, A61K 31/19, A61K 35/33, A61K 45/06, A61K 47/10, A61K 47/36, A61K 9/00, A61P 25/00

(54) **COMBINATION OF CANNABIDIOL AND FIBROBLASTS FOR USE IN THE TREATMENT OF DISC DEGENERATIVE DISEASE**
KOMBINATION AUS CANNABIDIOL UND FIBROBLASTEN ZUR VERWENDUNG BEI DER BEHANDLUNG VON BANDSCHEIBENDEGENERATION
COMBINAISON DE CANNABIDIOL ET DE FIBROBLASTES DESTINÉE AU TRAITEMENT DES MALADIES DÉGÉNÉRATIVES DISCALES

(30) Priority: 13.10.2019 US 201962914523 P
(43) Date of publication of application: 17.08.2022
(73) Proprietor: FibroBiologics, Inc., Houston, Texas 77289 (US)
(72) Inventor: O'HEERON, Pete, Houston, Texas 77059 (US); ICHIM, Thomas, Houston, Texas 77058 (US)
(74) Representative: J A Kemp LLP
(86) International application number: PCT/US2020/055313
(87) International publication number: WO 2021/076465

(56) References cited:
- US-A1- 2014 234 433
- US-A1- 2014 314 726
- CHEN JIE ET AL: "Protective effect of cannabidiol on hydrogen peroxide-induced apoptosis, inflammation and oxidative stress in nucleus pulposus cells", MOLECULAR MEDICINE REPORTS, vol. 14, no. 3, 13 July 2016 (2016-07-13), GR, pages 2321 - 2327, XP093071326, ISSN: 1791-2997, DOI: 10.3892/mmr.2016.5513
- RAWAL ET AL., EFFECT OF CANNABIDIOL ON HUMAN GINGIVAL FIBROBLAST EXTRACELLULAR MATRIX METABOLISM: MMP PRODUCTION AND ACTIVITY, AND PRODUCTION OF FIBRONECTIN AND TRANSFORMING GROWTH FACTOR BETA, 10 October 2011 (2011-10-10), XP055819119, Retrieved from the Internet <URL:https://europepmc.org/article/med/22092062> [retrieved on 20201210]
- SILVEIRA ET AL., PROTECTIVE EFFECTS OF CANNABIDIOL ON LESION-INDUCED INTERVERTEBRAL DISC DEGENERATION, 17 December 2014 (2014-12-17), XP055819122, Retrieved from the Internet <URL:https://www.ncbi.nlm.nih.gov/pmc/articles/PMC4269422/pdf/pone.0113161.pdf> [retrieved on 20201210]

## Description

### TECHNICAL FIELD

Described herein is cannabidiol and at least one therapeutic intervention comprising a therapeutically effective amount of fibroblast cells for use in a method of treating a degenerative disc disease in an individual.

### BACKGROUND

Lower back pain, at least in a large proportion of patients, is caused by an inflammatory event that occurs in conjunction with lumbar disc degeneration. The link between inflammation and pain is established in studies showing that radiologically degenerated discs are not associated with pain in a large number of subjects. In contrast, the presence of localized inflammation can be associated with disc degeneration, as exemplified by the presence of granulation tissue which is believed to be causative of nociception and the symptoms of chronic, intractable, lower back pain (1,2).

Although disc degeneration continues to have a tremendous and ever-increasing impact worldwide, current treatment options do not address the underlying cause. Current treatments include bed rest, nonsteroidal anti-inflammatory medication in the early phases of pathology, and procedures such as discectomy, arthroplasty (joint replacement), injection of artificial nucleus pulposus and fusion in the later phases when the prior approaches did not ameliorate pain. Approaches such as those previously mentioned not only are unpredictable, but also deal almost exclusively with end-stage clinical manifestations, and therefore do nothing to alter the disease process itself. Additionally, procedures such as vertebral fusion result in the increased incidence of disc degeneration in the adjacent discs due to alterations in the biomechanical distribution of work-load.

Recent advances in both biotechnology and our understanding of the biochemical makeup and environment of the intervertebral disc have led to increased interest in the process of degeneration and the possibility of developing novel treatments aimed directly at disc preservation. Certain genes found to have significant impact on matrix synthesis and catabolism within the disc have provided targets for scientists seeking to alter the balance between the two. To this end, much attention over the past several years has centered on gene therapy, and these efforts have yielded promising preclinical results with regard to its use in treating disc degeneration (3). Unfortunately, none of these approaches are near clinical implementation at the time of writing. Additionally, it is important to note that even in the circumstance that disc regeneration alone can be achieved through gene-therapy or other interventional means, the underlying process that originally caused the degeneration must be addressed in order to prevent re-occurrence.

Currently, no biologic treatment is widely available for disc degeneration. However, many different molecules of potential therapeutic benefit are being investigated. The focus of molecular therapy has been to prevent or reverse one or more aspects of these changes in the disc extracellular matrix. At least four different classes of molecules may be effective in disc repair. These include anticatabolics, mitogens, chondrogenic morphogens and intracellular regulators (4-6).

Hallmarks of disc degeneration include loss of proteoglycan, water, and Type II collagen in the disc matrix. Furthermore, qualitative changes in the matrix are less well defined, including loss of the higher molecular weight proteoglycans, and other changes that are more difficult to quantify (collagen cross-linking, organization of the proteoglycan, etc). An important process in disc degeneration seems to be the change of the differentiated chondrocyte phenotype in the nucleus pulposus into a more fibrotic phenotype. Together these changes in the disc matrix lead to alteration of the disc and vertebral anatomy that ultimately is associated with a pathologic condition (7,8) .

Because matrix loss is an imbalance between matrix synthesis and degradation, it is possible to increase disc matrix by increasing synthesis or by decreasing degradation. One approach is to prevent matrix loss by inhibiting the degradative enzymes.

Degenerated discs have elevated concentrations of matrix metalloproteinases (MMPs). Within the matrix, MMP activity is normally inhibited by tissue inhibitors of MMPs (TIMPs) (9-11). Wallach et al tested whether one of these anticatabolic molecules, TIMP-1, could increase the accumulation of matrix proteoglycans with in vitro experiments. The researchers found that indeed TIMP-1 expression in disc cells increased accumulation and also increased the "measured synthesis rate" of proteoglycans (11). Inflammatory processes are well known to be associated with degenerative disc disease. Studies have shown elevations of inflammatory cytokines such as interleukin-1 (12-16); TNF-alpha (17-20); and interleukin-6 (21), in patients with degenerating discs. The inflammatory cytokines serve to accelerate degeneration by several mechanisms including stimulation of matrix metalloproteases (22,23), which destroy the extracellular matrix (17,24), as well as stimulation of nitric oxide production, which causes apoptosis of nucleus pulposus cells (25,26). It is interesting that cells in the degenerating disc not only produce inflammatory cytokines but also are hypersensitive towards inflammatory cytokines in terms of producing higher amounts of inflammatory cytokines in response to other inflammatory cytokines. This suggests the existence of self-amplifying feedback loops (27). The impact of inflammatory cytokines on production of extracellular matrix proteins is also believed to contribute to degeneration in part by reducing regenerative activities (28). For example, in one study, researchers generated in vitro formed nucleus pulposus tissues, which they then treated with the inflammatory cytokine TNF-alpha (up to 50 ng/mL) over 48 hours. Tissues were assessed for histologic appearance, proteoglycan and collagen contents, as well as proteoglycan and collagen synthesis. Reverse transcriptase polymerase chain reaction was used to determine the effect of TNF-alpha on NP cell gene expression. Proteoglycan degradation was assessed by immunoblot analysis. At doses of 1-5 ng/mL, TNF-alpha induced multiple cellular responses, including: decreased expression of both aggrecan and type II collagen genes; decreases in the accumulation and overall synthesis of aggrecan and collagen; increased expression of MMP-1, MMP-3, MMP-13, ADAM-TS4, and ADAM-TS5; and induction of ADAM-TS dependent proteoglycan degradation. Within 48 hours, these cellular responses resulted in NP tissue with only 25% of its original proteoglycan content. These results strongly support the ability of TNF-alpha to potently inhibit function of nucleus pulposus cells and block ability to regenerate (29).

The importance of inflammatory cytokines in degenerative disc disease is highlighted not only by their direct causing of physical pathology but also studies showing effects at directly inducing pain. For example, Harii et al used retrograde neurotracing and immunohistochemistry to investigate the effect of the TNF-alpha inhibitor, etanercept, on a molecular model of pain, the calcitonin gene-related peptide (CGRP) expression in dorsal root ganglion (DRG) neurons innervating intervertebral discs in rats. The idea was that degeneration of lumbar intervertebral discs is a cause of lower back pain. Potentially, TNF-α in the intervertebral disc is a major contributor to discogenie pain. Effects of TNF-α inhibition on CGRP expression in DRG neurons were evaluated. FluoroGold-labeled neurons innervating the L4/5 disc were distributed throughout L1-L6 DRGs in all groups. Of the FluoroGold-labeled neurons, the proportion of CGRP-immunoreactive neurons was 21% ± 4% in the sham surgery control group, 32% ± 7% in the puncture + saline group, and 23% ± 4% in the puncture + etanercept group. The proportion of CGRP-immunoreactive neurons was significantly greater in the puncture + saline group compared with the sham control and puncture + etanercept groups. The authors concluded that in this model, CGRP was upregulated in DRG neurons innervating damaged discs and that direct intradiscal application of etanercept immediately after disc puncture suppressed CGRP expression in DRG neurons innervating injured discs (30). Other studies have reported that TNF-alpha, as well as other inflammatory cytokines such as IL-1 (31), are associated with induction of direct pain (32-43). Chen et al 2016 (Molecular Medicine Reports, 2016 September, Vol. 14, No.3, pages 2321-7) describes the protective effect of cannabidiol on hydrogen peroxide-induced apoptosis, inflammation and oxidative stress in nucleus pulposus cells. Silveira et al. (PLoS One 9.12 (2014): e113161) relates to the protective effects of cannabidiol on lesion-induced intervertebral disc degeneration.

Regenerative cells, including mesenchymal stem cells, fibroblasts, hematopoietic stem cells, and even immune cells, all require a proper environment when implanted in order to perform their healing functions. US 2014/314726 A1 describes the use of fibroblasts for the treatment of degenerative disc disease. The present disclosure provides the utilization of cannabidiol as a means of reducing inflammation and stimulating efficacy of regenerative cells and/or growth factors when administered to patients with degenerative disc disease.

### SUMMARY OF THE INVENTION

The present invention provides cannabidiol for use in a method of treating a degenerative disc disease in an individual, said method comprising administering the cannabidiol and at least one therapeutic intervention for a degenerative disc disease to an individual at the same time, and said therapeutic intervention comprising a therapeutically effective amount of fibroblast cells.

The cannabidiol may be administered systemically and/or locally, such as into a disc, to the individual. The administration of cannabidiol may be administered to the individual at a dosage and frequency sufficient to reduce apoptosis of cells in the nucleus pulposus, including endogenous and/or exogenous cells in the nucleus pulposus. The exogenous cells in the nucleus pulposus may comprise the therapeutic intervention for a degenerative disc disease encompassed herein. The endogenous cells in the nucleus pulposus may comprise chondrocytes, notochord cells, notochord progenitor cells, chondrocyte progenitor cells, or a combination thereof. The cannabidiol may be administered at any dosage required for a therapeutic effect, including a dosage between 50 mg to 500 mg, or any range derivable therein, to the individual daily. The cannabidiol may be administered at any frequency required for a therapeutic effect, including administered daily, twice daily, weekly, bi-weekly, monthly, bi-monthly, and so forth. The cannabidiol may comprise or consist of (-)-cannabidiol. The cannabidiol may comprise a gel formulation, which may comprise a permeation-enhanced gel. In some aspects, the cannabidiol may be administered with a localization composition, which may comprise one or more extracellular matrix molecules, including hyaluronic acid and/or long chain hyaluronic acid molecules.

The fibroblast cells encompassed herein, including fibroblasts administered or not administered to an individual, may or may not be manipulated. In some embodiments, the fibroblasts are contacted with hCG, oxytocin, or a combination thereof, which augments immune modulatory activity, in at least specific cases. The hCG may be contacted or administered to cells at a concentration between 1 nM to 1 µM, or 10 nM to 100 nM, or any range derivable therein, per million fibroblasts. The oxytocin may be contacted or administered to cells at a concentration between 1 nM to 10 µM, or 100 nM to 1 µM, or any range derivable therein. The fibroblasts may be from one or more tissues selected from the group consisting of adipose tissue, dermal tissue, bone marrow, peripheral blood, Wharton's Jelly, placenta, omentum, mobilized peripheral blood, and a combination thereof. The fibroblasts encompassed herein may proliferate at a rate of 14-21 hours per cell multiplication. The fibroblasts encompassed herein may secrete 0.1 pg-77 pg of interleukin-1 per culture of 1 million fibroblasts at 75% confluence on a surface. The fibroblasts encompassed herein may secrete 1 pg-500 pg of FGF-1 per culture of 1 million fibroblasts at 75% confluence on a surface. In some embodiments, the fibroblasts may substantially decrease, such as by approximately more than 20%, the ability of responding T cells to proliferate in a mixed lymphocyte reaction.

### BRIEF DESCRIPTION OF THE DRAWINGS

For a more complete understanding of the present disclosure, reference is now made to the following descriptions taken in conjunction with the accompanying drawing, in which:

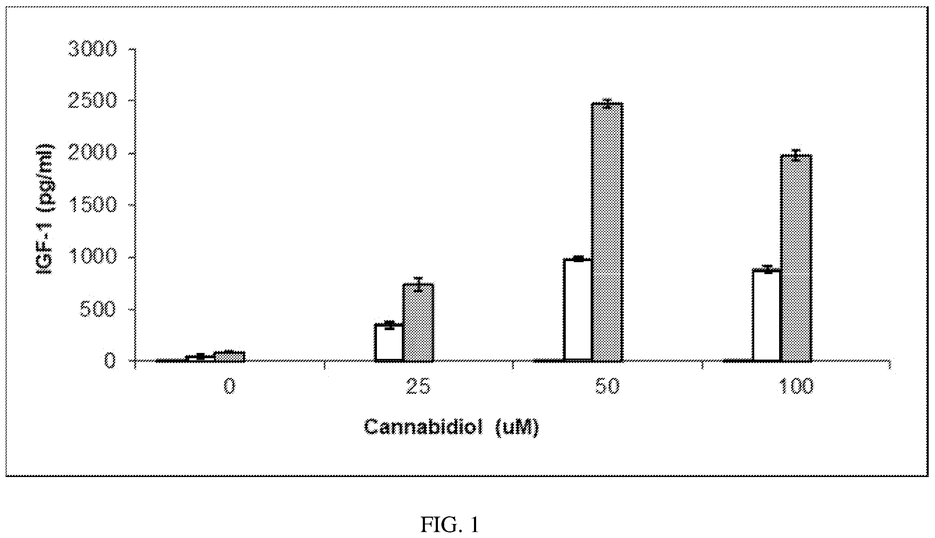

FIG. 1 shows stimulation of IGF-1 from fibroblasts by CBD at various concentrations. In the groupings of three bars, the middle bar at each concentration represents 100,000 fibroblasts co-cultured with CBD at the indicated concentration. The right bar at each concentration represents 200,000 fibroblasts co-cultured with CBD at the indicated concentration. The left bar at each concentration represents 0 fibroblasts co-cultured with CBD at the indicated concentration;

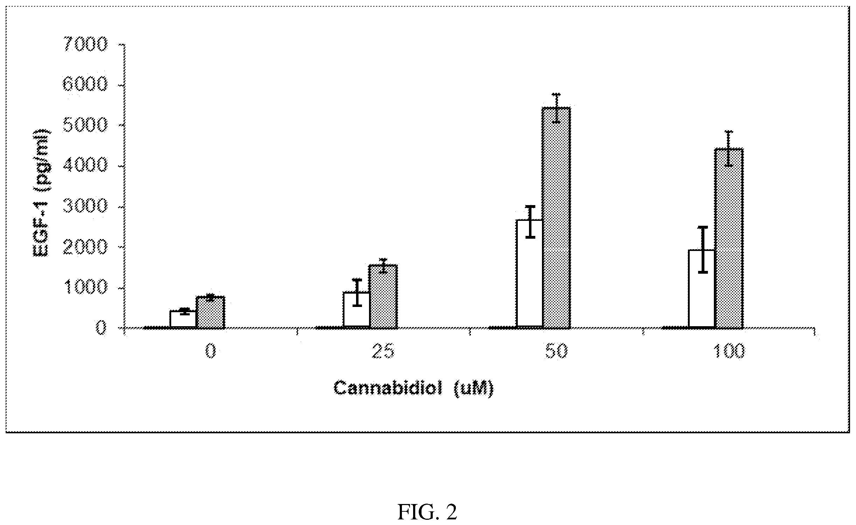

FIG. 2 shows stimulation of EGF-1 from fibroblasts by CBD at various concentrations. In the groupings of three bars, the middle bar at each concentration represents 100,000 fibroblasts co-cultured with CBD at the indicated concentration. The right bar at each concentration represents 200,000 fibroblasts co-cultured with CBD at the indicated concentration. The left bar at each concentration represents 0 fibroblasts co-cultured with CBD at the indicated concentration.

### DETAILED DESCRIPTION

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

References to the methods of treatment by therapy or surgery or *in vivo* diagnosis methods in the description are to be interpreted as references to compounds, pharmaceutical compositions and medicaments of the present invention for use in those methods.

In keeping with long-standing patent law convention, the words "a" and "an" when used in the present specification in concert with the word comprising, including the claims, denote "one or more." Some aspects of the disclosure may consist of or consist essentially of one or more elements, method steps, and/or methods of the disclosure.

Throughout this specification, unless the context requires otherwise, the words "comprise", "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

Reference throughout this specification to "one aspect," "an aspect," "a particular aspect," "a related aspect," "a certain aspect," "an additional aspect," or "a further aspect" or combinations thereof means that a particular feature, structure or characteristic described in connection with the aspect is included in at least one aspect of the present disclosure. Thus, the appearances of the foregoing phrases in various places throughout this specification are not necessarily all referring to the same aspect. Furthermore, the particular features, structures, or characteristics may be combined in any suitable manner in one or more aspects.

### I. Cannabidiol

As used herein, "cannabidiol" or "CBD" refers to cannabidiol; pharmaceutically acceptable salts of cannabidiol, CBD includes, 2-[3-methyl-6-(1-methylethenyl)-2-cyclohexen-1-yl]-5-pentyl-1,3-benzenediol as well as to pharmaceutically acceptable salts, The synthesis of CBD is described, for example, in Petilka et al., Helv. Chim. Acta, 52:1102 (1969) and in Mechoulam et al., J. Am. Chem. Soc., 87:3273 (1965).

Certain aspects of the disclosure concern the use of cannabidiol (CBD), including as an adjuvant to regenerative cell activity or regenerative cell therapy. In particular aspects, cannabidiol is administered together with regenerative cells for the treatment of disc degeneration or a degenerative disc disease. Administration of cannabidiol may be performed systemically and/or locally. In some aspects, CBD is utilized to activate regenerative cells *in vitro* or *ex vivo* prior to implantation. In particular aspects, CBD is administered systemically, which may increase suitability of the microenvironment for sustaining regenerative cell activity, suppress inflammation, and/or directly stimulate regenerative cells to perform healing functions.

The CBD may or may not be synthetic CBD. The CBD may be purified CBD. The CBD may be botanically derived. CBD may be administered transdermally on the subject's upper arm and shoulder. In some aspects, the CBD is administered transdermally on the subject's thigh or back. The CBD may be administered directly to a disc, whether or not it is showing signs of degeneration. Locally administering an effective amount of cannabidiol (CBD) in specific cases can reduce an intensity of at least one adverse event or side effect relative to orally administering CBD. The at least one adverse event or side effect may be a gastrointestinal (GI) adverse event. The at least one adverse event or side effect can be an adverse event or side effect affecting liver function. In some aspects, the at least one adverse event is somnolence. In some aspects, the frequency and intensity of somnolence is reduced as an adverse event by transdermal administration.

In some aspects, the CBD comprises or consists of (-)-CBD. An effective amount, such as a therapeutically effective amount, of CBD may be between about 50 mg to about 500 mg daily. In some aspects, the effective amount of CBD, which may be administered to an individual, is initiated at about 50 mg daily and titrated up to about 500 mg daily. In some aspects, the effective amount of CBD, which may be administered to an individual, initiated at about 50 mg daily and titrated up to about 250 mg daily. In some aspects, the effective amount of CBD, which may be administered to an individual, is initiated at 250 mg daily. In some aspects, the effective amount of CBD, which may be administered to an individual, is initiated at 500 mg daily. In some aspects, the 500 mg daily dose is administered to patients that weigh greater than 35 kg. The CBD may be administered at a frequency useful for a therapeutic effect, including in a single daily dose or in two daily doses. In some aspects, the effective amount of CBD can be 390 mg in divided daily doses.

As used herein, the term "transdermally" when in the context of administration, refers to contacting the CBD with the patient's or subject's skin under conditions effective for the CBD to penetrate the skin. In particular aspects, the CBD is formulated as a gel or an oil. In some aspects, the CBD is formulated as a permeation-enhanced gel. The gel may contain between 1% (wt/wt) CBD to 7.5% (wt/wt) CBD. In some aspects, the gel contains 4.2% (wt/wt) CBD. In some aspects, the gel contains 7.5% (wt/wt) CBD. In some aspects, the transdermal preparation can be a cream, a salve or an ointment. The CBD can be delivered by a bandage, pad or patch. The CBD can be in a gel form and can be pharmaceutically-produced as a clear, permeation-enhanced gel that is designed to provide controlled drug delivery transdermally with once- or twice-daily dosing. The CBD gel can between 1% (wt/wt) CBD to 7.5% (wt/wt) CBD, or any range derivable therein. The CBD gel can have, for example, 4.2% (wt/wt) CBD or 7.5% (wt/wt) CBD). The CBD gel can be applied topically by the patient or caregiver to the patient's upper arm and shoulder, back, thigh, or any combination thereof. The CBD gel can include diluents and carriers as well as other conventional excipients, such as wetting agents, preservatives, and suspending and dispersing agents.

The CBD gel can comprise a solubilizing agent, a permeation enhancer, a solubilizer, antioxidant, bulking agent, thickening agent, and/or a pH modifier. The composition of the CBD gel can be, for example, cannabidiol present in an amount of about 0.1% to about 20% (wt/wt) of the composition; a lower alcohol having between 1 and 6 carbon atoms present in an amount of about 15% to about 95% (wt/wt) of the composition; a first penetration enhancer present in an amount of about 0.1% to about 20% (wt/wt) of the composition; and water in a quantity sufficient for the composition to total 100% (wt/wt). Other formulations of the CBD gel can be found in International Publication No. WO 2010/127033.

In some aspects, a cannabinoid, such as cannabidiol or a prodrug of cannabidiol, is delivered systemically to achieve therapeutically effective plasma concentrations in an individual. However, cannabinoid oral dosage forms, including those comprising cannabidiol, must overcome several obstacles in order to achieve a therapeutically-effective systemic concentration. First, cannabinoids are generally highly lipophilic. Their limited water solubility thereby restricts the amount of cannabinoid available for absorption in the gastrointestinal tract. Second, cannabidiol, as with the other cannabinoids, undergoes substantial first-pass metabolism when absorbed from the human gastrointestinal tract. Finally, the oral bioavailability of any product is further diminished when a patient suffers from nausea or emesis, as either the patient avoids taking his oral medications or the oral dosage form does not remain in the gastrointestinal tract for a sufficient period of time to release the entire dose and achieve a therapeutic concentration. Therefore, in view of the foregoing, it would be desirable to systemically deliver therapeutically effective amounts of a cannabinoid, such as cannabidiol or cannabidiol prodrug, to a mammal in need thereof for the treatment of one or more medical conditions responsive to cannabinoids, including pain, degenerative disc disease, nausea or appetite stimulation, by a route of administration that does not depend upon absorption from the gastrointestinal tract of the mammal.

One non-oral route of administration for the systemic delivery of cannabidiol is transdermal administration. In addition, the epidermis and dermis of many mammals, such as humans and guinea pigs, contains enzymes which are capable of metabolizing active pharmaceutical agents which pass through the stratum corneum. The metabolic process occurring in the skin of mammals, such as humans, can be utilized to deliver pharmaceutically effective quantities of a cannabinoid, such as cannabidiol, to the systemic circulation of a mammal in need thereof. Encompassed herein are prodrugs of cannabinoids, such as cannabidiol prodrugs, and compositions comprising prodrugs of cannabinoids that can be transdermally administered to a mammal, such as a human, so that the metabolic product resulting from metabolism in the skin is the cannabinoid which is systemically available for the treatment of a medical condition responsive to cannabinoid. Unfortunately, due to its highly lipophilic nature, cannabidiol is poorly absorbed through membranes such as the skin of mammals, including humans. Therefore, the success of transdermally administering therapeutically effective quantities of cannabidiol to a mammal in need thereof within a reasonable time frame and over a suitable surface area has been substantially limited.

In some aspects, cannabidiol is administered to an individual locally, for example, administered to a disc of the individual. The cannabidiol may be administered locally to the individual at any dosage and frequency, including dosages and frequencies encompassed herein. The locally-administered cannabidiol may be administered with one or more other compositions, including the cellular therapies encompassed herein. In some aspects, the cannabidiol administered to a disc in the individual reduces apoptosis of cells comprising the disc of the individual. The cells comprising the disc may be endogenous (including notochord cells, notochord progenitor cells, chondrocytes, and/or chondrocyte progenitor cells) and/or exogenous cells, including exogenous cells comprising any cellular therapy administered to the individual.

In some aspects, administration of cannabidiol to an individual are performed together with other agents that inhibit inflammation. In one aspect, administration of interleukin-1 (IL-1) receptor antagonist, including as a protein, a gene, and/or a mRNA, is performed to reducing some aspects of inflammation, which synergizes unexpectedly with administration of cannabidiol.

In some aspects, cannabidiol or cannabidiol with one or more therapeutic interventions for a degenerative disc disease is administered with a localization composition. The localization composition may be one or more extracellular matrix molecules, including hyaluronic acid and/or long chained hyaluronic acid.

In some aspects, cannabidiol is contacted with, cultured with, or administered to cells such as fibroblasts, which may augment regenerative activity in the cells. Cells, including fibroblasts, contacted with, cultured with, or administered cannabidiol (including cannabidiol comprising or consisting of (-)-cannabidiol) may increase the production of IGF-1 and/or EGF-1. The fibroblasts contacted with, cultured with, or administered cannabidiol (including cannabidiol comprising or consisting of (-)-cannabidiol) may be used for methods encompassed herein. The fibroblasts contacted with, cultured with, or administered cannabidiol (including cannabidiol comprising or consisting of (-)-cannabidiol) may be administered locally (including intradiscally) and/or systemically to an individual, including an individual having, or at risk of having, a degenerative disc disease.

### II. Therapeutic Intervention for a Degenerative Disc Disease

The disclosure concerns the use of one or more therapeutic interventions for a degenerative disc disease, which includes fibroblasts. In particular, fibroblasts together with cannabidiol are administered to an individual. Fibroblasts may be from any autologous, allogenic, or xenogeneic source. Fibroblasts may be from tissue selected from the group consisting of adipose tissue, dermal tissue, bone marrow, peripheral blood, Wharton's Jelly, placenta, omentum, mobilized peripheral blood, and a combination thereof.

In certain aspects, fibroblasts, including manipulated or un-manipulated fibroblasts, are delivered locally, such as to a disc, to an individual, such as an individual in need of treatment for a degenerative disc disease. The fibroblasts may be administered as a formulation including a formulation comprising a hydrogel. The hydrogel may be comprised of, for example, platelet rich plasma (PRP) and hyaluronic acid (HA) blended with batroxobin (BTX) as gelling agent. The fibroblasts may be encapsulated in PRP/HA/BTX hydrogel and cultured in both growing medium and medium with or without TGF-β1 up to day 21. In one aspect, the hydrogel jellifies in 15 minutes 20 °C and in 3 minutes at 37 °C in a manner such that said fibroblasts maintain high cell viability and proliferation. In one aspect, fibroblasts are used for intra-disc injections in patients with degenerative disc disease. In such an aspect, said fibroblasts are cultured in means to enhance GAG production, which may be achieved by culture with cytokines such as TGF-beta. Methodologies for growth of mesenchymal stem cells, which may be modified for fibroblast-specific use are provided in the following, which is incorporated by reference (44).

Certain aspects concern the activation of fibroblasts prior to therapeutic use, and/or administration of agents which act as "regenerative adjuvants" for the fibroblasts. The cells in the formulation may display typical fibroblast morphologies when growing in cultured monolayers. Specifically, cells may display an elongated, fusiform or spindle appearance with slender extensions, or cells may appear as larger, flattened stellate cells which may have cytoplasmic leading edges. A mixture of these morphologies may also be observed. The cells may express proteins characteristic of normal fibroblasts including the fibroblast-specific marker, CD90 (Thy-1), a 35 kDa cell-surface glycoprotein, and the extracellular matrix protein, collagen. The fibroblast dosage formulation may be an autologous cell therapy product composed of a suspension of autologous fibroblasts, grown from a biopsy of each individual's own skin using standard tissue culture procedures. In particular aspects, the fibroblasts can also be used to create other cell types for tissue repair or regeneration.

The fibroblasts encompassed herein may be generated by outgrowth from a biopsy of skin of an individual receiving administrations encompassed herein (in the case of autologous preparations), or skin of healthy donors (for allogeneic preparations). Skin-derived fibroblasts are disclosed as an example, however fibroblasts encompassed in the disclosure may be from any source or tissue. In some aspects, fibroblasts are used from young donors. In another aspect, fibroblasts are transfected with genes to allow for enhanced growth and overcoming of the Hayflick limit. Subsequent to derivation of cells expansion in culture using standard cell culture techniques. Said techniques include expansion in liquid media, utilization of media such as RPMI 1640, DMEM, OPTI-MEM, or AIM-V. Standard techniques for tissue culture are well known in the art and include the need for dissociating cells. In some aspects, dissociation involves treatment with trypsin or collagen to allow for cell passaging. Skin tissue (dermis and epidermis layers) may be biopsied from a subject's post-auricular area. In one aspect, disclosed for the purpose of describing a non-limiting example, the starting material is composed of three 3-mm punch skin biopsies collected using standard aseptic practices. The biopsies are collected by a skilled artisan, placed into a vial containing sterile phosphate buffered saline (PBS). The biopsies are shipped in a 2-8 °C. refrigerated shipper back to the manufacturing facility. In one aspect, after arrival at the manufacturing facility, the biopsy is inspected and, upon acceptance, transferred directly to the manufacturing area. Upon initiation of the process, the biopsy tissue is then washed prior to enzymatic digestion. After washing, a Liberase Digestive Enzyme Solution is added without mincing, and the biopsy tissue is incubated at 37.0 +/- 2.0 °C. for one hour. Time of biopsy tissue digestion is a critical process parameter that can affect the viability and growth rate of cells in culture. Liberase is a collagenase/neutral protease enzyme cocktail obtained formulated from Lonza Walkersville, Inc. (Walkersville, Md.) and unformulated from Roche Diagnostics Corp. (Indianapolis, Ind.). Alternatively, other commercially available collagenases may be used, such as Serva Collagenase NB6 (Helidelburg, Germany). After digestion, Initiation Growth Media (IMDM, GA, 10% Fetal Bovine Serum (FBS)) is added to neutralize the enzyme, cells are pelleted by centrifugation and resuspended in 5.0 mL Initiation Growth Media. Alternatively, centrifugation is not performed, with full inactivation of the enzyme occurring by the addition of Initiation Growth Media only. Initiation Growth Media is added prior to seeding of the cell suspension into a T-175 cell culture flask for initiation of cell growth and expansion. A T-75, T-150, T-185 or T-225 flask can be used in place of the T-75 flask. Cells are incubated at 37 +/- 2.0 °C. with 5.0 +/- 1.0% CO₂ and fed with fresh Complete Growth Media approximately every three to five days. All feeds in the process are performed by removing half of the Complete Growth Media and replacing the same volume with fresh media. Alternatively, full feeds can be performed. Cells should not remain in the T-175 flask greater than approximately 30 days prior to passaging. Confluence is monitored throughout the process to ensure adequate seeding densities during culture splitting. When cell confluence is greater than or equal to 40% in the T-175 flask, they are passaged by removing the spent media, washing the cells, and treating with Trypsin-EDTA to release adherent cells in the flask into the solution. Cells are then trypsinized and seeded into a T-500 flask for continued cell expansion. Alternately, one or two T-300 flasks, One Layer Cell Stack (1 CS), One Layer Cell Factory (1 CF) or a Two Layer Cell Stack (2 CS) can be used in place of the T-500 Flask.

Morphology is evaluated at each passage and prior to harvest to monitor the culture purity throughout the culture purity throughout the process. Morphology is evaluated by comparing the observed sample with visual standards for morphology examination of cell cultures. The cells display typical fibroblast morphologies when growing in cultured monolayers. Cells may display either an elongated, fusiform or spindle appearance with slender extensions, or appear as larger, flattened stellate cells which may have cytoplasmic leading edges. A mixture of these morphologies may also be observed. Fibroblasts in less confluent areas can be similarly shaped, but randomly oriented. The presence of keratinocytes in cell cultures is also evaluated. Keratinocytes appear round and irregularly shaped and, at higher confluence, they appear organized in a cobblestone formation. At lower confluence, keratinocytes are observable in small colonies. Cells are incubated at 37 +/- 2.0 °C. with 5.0 +/- 1.0% CO2 and passaged every three to five days in the T-500 flask and every five to seven days in the ten layer cell stack (10CS). Cells should not remain in the T-500 flask for more than 10 days prior to passaging. Quality Control (QC) release testing for safety of the Bulk Drug Substance (cells) includes sterility and endotoxin testing. When cell confluence in the T-500 flask is >95%, cells are passaged to a 10 CS culture vessel. Alternately, two Five Layer Cell Stacks (5 CS) or a 10 Layer Cell Factory (10 CF) can be used in place of the 10 CS. Passage to the 10 CS is performed by removing the spent media, washing the cells, and treating with Trypsin-EDTA to release adherent cells in the flask into the solution. Cells are then transferred to the 10 CS. Additional Complete Growth Media is added to neutralize the trypsin and the cells from the T-500 flask are pipetted into a 2 L bottle containing fresh Complete Growth Media. The contents of the 2 L bottle are transferred into the 10 CS and seeded across all layers. Cells are then incubated at 37 +/- 2.0 °C. with 5.0 +/- 1.0% CO₂ and fed with fresh Complete Growth Media every five to seven days. Cells should not remain in the 10CS for more than 20 days prior to passaging. In one aspect, the passaged dermal fibroblasts are rendered substantially free of immunogenic proteins present in the culture medium by incubating the expanded fibroblasts for a period of time in protein free medium, Primary Harvest When cell confluence in the 10 CS is 95% or more, cells are harvested. Harvesting is performed by removing the spent media, washing the cells, treating with Trypsin-EDTA to release adherent cells into the solution, and adding additional Complete Growth Media to neutralize the trypsin. Cells are collected by centrifugation, resuspended, and in-process QC testing performed to determine total viable cell count and cell viability.

In some aspects, when large numbers of cells are required after receiving cell count results from the primary 10 CS harvest, an additional passage into multiple cell stacks (up to four 10 CS) is performed. For additional passaging, cells from the primary harvest are added to a 2 L media bottle containing fresh Complete Growth Media. Resuspended cells are added to multiple cell stacks and incubated at 37 +/- 2.0 °C. with 5.0 +/- 1.0% CO₂. The cell stacks are fed and harvested as described above, except cell confluence must be 80% or higher prior to cell harvest. The harvest procedure is the same as described for the primary harvest above. A mycoplasma sample from cells and spent media is collected, and cell count and viability performed as described for the primary harvest above. The method decreases or eliminates immunogenic proteins by avoiding their introduction from animal-sourced reagents. To reduce process residuals, cells are cryopreserved in protein-free freeze media, then thawed and washed prior to prepping the final injection to further reduce remaining residuals. If additional Drug Substance is needed after the harvest and cryopreservation of cells from additional passaging is complete, aliquots of frozen Drug Substance--Cryovial are thawed and used to seed 5 CS or 10 CS culture vessels. Alternatively, a four layer cell factory (4 CF), two 4 CF, or two 5 CS can be used in place of a 5 CS or 10 CS. A frozen cryovial(s) of cells is thawed, washed, added to a 2 L media bottle containing fresh Complete Growth Media and cultured, harvested and cryopreserved as described above. The cell suspension is added Cell confluence must be 80% or more prior to cell harvest.

At the completion of culture expansion, the cells are harvested and washed, then formulated to contain 100,000 cells to 2.7 x 10⁷ cells/mL, or more, with a target of 2.2x 10⁷ cells/mL. Alternatively, the target can be adjusted within the formulation range to accommodate different indication doses. The drug substance comprises a population of viable, autologous human fibroblast cells suspended in a cryopreservation medium consisting of Iscove's Modified Dulbecco's Medium (IMDM) and Profreeze-CDM^{™} (Lonza, Walkerville, Md.) plus 7.5% dimethyl sulfoxide (DMSO). Alternatively, a lower DMSO concentration may be used in place of 7.5% or CryoStor^{™} CS5 or CryoStor^{™} CS10 (BioLife Solutions, Bothell, Wash.) may be used in place of IMDM/Profreeze/DMSO. In addition to cell count and viability, purity/identity of the Drug Substance is performed and must confirm the suspension contains 98% or more fibroblasts. The usual cell contaminants include keratinocytes. The purity/identify assay employs fluorescent-tagged antibodies against CD90 and CD 104 (cell surface markers for fibroblast and keratinocyte cells, respectively) to quantify the percent purity of a fibroblast cell population. CD90 (Thy-1) is a 35 kDa cell-surface glycoprotein. Antibodies against CD90 protein have been shown to exhibit high specificity to human fibroblast cells. CD104, integrin β4 chain, is a 205 kDa transmembrane glycoprotein which associates with integrin α6 chain (CD49f) to form the α6/ β4 complex. This complex has been shown to act as a molecular marker for keratinocyte cells (Adams and Watt 1991).

Antibodies to CD 104 protein bind to 100% of human keratinocyte cells. Cell count and viability is determined by incubating the samples with Viacount Dye Reagent and analyzing samples using the Guava PCA system. The reagent is composed of two dyes, a membrane-permeable dye which stains all nucleated cells, and a membrane-impermeable dye which stains only damaged or dying cells. The use of this dye combination enables the Guava PCA system to estimate the total number of cells present in the sample, and to determine which cells are viable, apoptotic, or dead. The method was custom developed specifically for use in determining purity/identity of autologous cultured fibroblasts.

Alternatively, cells can be passaged from either the T-175 flask (or alternatives) or the T-500 flask (or alternatives) into a spinner flask containing microcarriers as the cell growth surface. Microcarriers are small bead-like structures that are used as a growth surface for anchorage-dependent cells in suspension culture. They are designed to produce large cell yields in small volumes. In this apparatus, a volume of Complete Growth Media ranging from 50 mL-300 mL is added to a 500 mL, IL or 2 L sterile disposable spinner flask. Sterile microcarriers are added to the spinner flask. The culture is allowed to remain static or is placed on a stir plate at a low RPM (15-30 RRM) for a short period of time (1-24 hours) in a 37 +/- 2.0 °C. with 5.0 +/-1.0% CO₂ incubator to allow for adherence of cells to the carriers. After the attachment period, the speed of the spin plate is increased (30-120 RPM). Cells are fed with fresh Complete Growth Media every one to five days, or when media appears spent by color change. Cells are collected at regular intervals by sampling the microcarriers, isolating the cells and performing cell count and viability analysis. The concentration of cells per carrier is used to determine when to scale-up the culture. When enough cells are produced, cells are washed with PBS and harvested from the microcarriers using trypsin-EDTA and seeded back into the spinner flask in a larger amount of microcarriers and higher volume of Complete Growth Media (300 mL-2 L). Alternatively, additional microcarriers and Complete Growth Media can be added directly to the spinner flask containing the existing microcarrier culture, allowing for direct bead-to-bead transfer of cells without the use of trypsinization and reseeding. Alternatively, if enough cells are produced from the initial T-175 or T-500 flask, the cells can be directly seeded into the scale-up amount of microcarriers. After the attachment period, the speed of the spin plate is increased (30-120 RPM). Cells are fed with fresh Complete Growth Media every one to five days, or when media appears spent by color change. When the concentration reaches the desired cell count for the intended indication, the cells are washed with PBS and harvested using trypsin-EDTA. Microcarriers used within the disposable spinner flask may be made from poly blend such as BioNOC II^{®}. (Cesco Bioengineering, distributed by Bellco Biotechnology, Vineland, N.J.) and FibraCel^{®}. (New Brunswick Scientific, Edison, N.J.), gelatin, such as Cultispher-G (Percell Biolytica, Astrop, Sweden), cellulose, such as Cytopore^{™} (GE Healthcare, Piscataway, N.J.) or coated/uncoated polystyrene, such as 2D MicroHex^{™} (Nunc, Weisbaden, Germany), Cytodex^{®}. (GE Healthcare, Piscataway, N.J.) or Hy-Q Sphere^{™} (Thermo Scientific Hyclone, Logan, Utah).

In another aspect, cells can be processed on poly blend 2D microcarriers such as BioNOC II^{®}. and FibraCel^{®}. using an automatic bellow system, such as FibraStage^{™} (New Brunswick Scientific, Edison, N.J.) or BelloCell^{®}. (Cesco Bioengineering, distributed by Bellco Biotechnology, Vineland, N.J.) in place of the spinner flask apparatus. Cells from the T-175 (or alternatives) or T-500 flask (or alternatives) are passaged into a bellow bottle containing microcarriers with the appropriate amount of Complete Growth Media, and placed into the system. The system pumps media over the microcarriers to feed cells, and draws away media to allow for oxygenation in a repeating fixed cycle. Cells are monitored, fed, washed and harvested in the same sequence as described above. Alternatively, cells can be processed using automated systems. After digestion of the biopsy tissue or after the first passage is complete (T-175 flask or alternative), cells may be seeded into an automated device. One method is an Automated Cellular Expansion (ACE) system, which is a series of commercially available or custom fabricated components linked together to form a cell growth platform in which cells can be expanded without human intervention. Cells are expanded in a cell tower, consisting of a stack of disks capable of supporting anchorage-dependent cell attachment. The system automatically circulates media and performs trypsinization for harvest upon completion of the cell expansion stage.

Alternatively, the ACE system can be a scaled down, including as a single lot unit version comprised of a disposable component that consists of cell growth surface, delivery tubing, media and reagents, and a permanent base that houses mechanics and computer processing capabilities for heating/cooling, media transfer and execution of the automated programming cycle. Upon receipt, each sterile irradiated ACE disposable unit will be unwrapped from its packaging and loaded with media and reagents by hanging pre-filled bags and connecting the bags to the existing tubing via aseptic connectors. The process continues as follows: a) Inside a biological safety cabinet (BSC), a suspension of cells from a biopsy that has been enzymatically digested is introduced into the "pre-growth chamber" (small unit on top of the cell tower), which is already filled with Initiation Growth Media containing antibiotics. From the BSC, the disposable would be transferred to the permanent ACE unit already in place; b) After approximately three days, the cells within the pre-growth chamber are trypsinized and introduced into the cell tower itself, which is pre-filled with Complete Growth Media. Here, the "bubbling action" caused by CO₂ injection force the media to circulate at such a rate that the cells spiral downward and settle on the surface of the discs in an evenly distributed manner; c) For approximately seven days, the cells are allowed to multiply. At this time, confluence will be checked (method unknown at time of writing) to verify that culture is growing. Also at this time, the Complete Growth Media will be replaced with fresh Complete Growth Media. CGM will be replaced every seven days for three to four weeks. At the end of the culture period, the confluence is checked once more to verify that there is sufficient growth to possibly yield the desired quantity of cells for the intended treatment; d) If the culture is sufficiently confluent, it is harvested. The spent media (supernatant) is drained from the vessel. PBS will then is pumped into the vessel (to wash the media, FBS from the cells) and drained almost immediately. Trypsin-EDTA is pumped into the vessel to detach the cells from the growth surface. The trypsin/cell mixture is drained from the vessel and enter the spin separator. Cryopreservative is pumped into the vessel to rinse any residual cells from the surface of the discs, and be sent to the spin separator as well. The spin separator collects the cells and then evenly resuspend the cells in the shipping/injection medium. From the spin separator, the cells will be sent through an inline automated cell counting device or a sample collected for cell count and viability testing via laboratory analyses. Once a specific number of cells has been counted and the proper cell concentration has been reached, the harvested cells are delivered to a collection vial that can be removed to aliquot the samples for cryogenic freezing.

In some aspects, the fibroblasts are cultured with or administered hCG and/or oxytocin, which may augment immune modulatory activity. Fibroblasts may be cultured with or administered hCG at a concentration between 1 nM to 1 µM or 10 nM to 100 nM or any range derivable therein. Fibroblast may be cultured with or administered oxytocin at a concentration between 1 nm to 10 µM or between 100 nm to 1 µM or any range derivable therein.

### EXAMPLES

The following examples are included to demonstrate preferred aspects of the disclosure.

### EXAMPLE 1

### CANNABIDIOL STIMULATES FIBROBLAST PRODUCTION OF IGF-1 AND EGF-1

Dermal fibroblasts were obtained from Allcells, Inc. and cultured for 48 hours in the indicated concentrations of CBD. As seen in FIG. 1, augmentation of IGF-1 was observed. As seen in FIG. 2, augmentation of EGF-1 was observed. Cultures were performed in OPTI-MEM media with 10% fetal calf serum.

### REFERENCES

1. Hwang, G. J., Suh, J. S., Na, J. B., Lee, H. M., and Kim, N. H. (1997) Contrast enhancement pattern and frequency of previously unoperated lumbar discs on MRI. J Magn Reson Imaging 7, 575-578
2. Peng, B., Wu, W., Hou, S., Li, P., Zhang, C., and Yang, Y. (2005) The pathogenesis of discogenic low back pain. J Bone Joint Surg Br 87, 62-67
3. Levicoff, E. A., Gilbertson, L. G., and Kang, J. D. (2005) Gene therapy for disc repair. Spine J 5, 287S-296S
4. Yoon, S. T. (2005) Molecular therapy of the intervertebral disc. Spine J 5, 280S-286S
5. Masuda, K., Oegema, T. R., Jr., and An, H. S. (2004) Growth factors and treatment of intervertebral disc degeneration. Spine 29, 2757-2769
6. Shimer, A. L., Chadderdon, R. C., Gilbertson, L. G., and Kang, J. D. (2004) Gene therapy approaches for intervertebral disc degeneration. Spine 29, 2770-2778
7. Setton, L. A., and Chen, J. (2004) Cell mechanics and mechanobiology in the intervertebral disc. Spine 29, 2710-2723
8. Roughley, P. J. (2004) Biology of intervertebral disc aging and degeneration: involvement of the extracellular matrix. Spine 29, 2691-2699
9. Roberts, S., Caterson, B., Menage, J., Evans, E. H., Jaffray, D. C., and Eisenstein, S. M. (2000) Matrix metalloproteinases and aggrecanase: their role in disorders of the human intervertebral disc. Spine 25, 3005-3013
10. Nagase, H., and Woessner, J. F., Jr. (1999) Matrix metalloproteinases. J Biol Chem 274, 21491-21494
11. Wallach, C. J., Sobajima, S., Watanabe, Y., Kim, J. S., Georgescu, H. I., Robbins, P., Gilbertson, L. G., and Kang, J. D. (2003) Gene transfer of the catabolic inhibitor TIMP-1 increases measured proteoglycans in cells from degenerated human intervertebral discs. Spine 28, 2331-2337
12. Solovieva, S., Leino-Arjas, P., Saarela, J., Luoma, K., Raininko, R., and Riihimaki, H. (2004) Possible association of interleukin 1 gene locus polymorphisms with low back pain. Pain 109, 8-19
13. Le Maitre, C. L., Freemont, A. J., and Hoyland, J. A. (2005) The role of interleukin-1 in the pathogenesis of human intervertebral disc degeneration. Arthritis Res Ther 7, R732-745
14. Le Maitre, C. L., Hoyland, J. A., and Freemont, A. J. (2007) Interleukin-1 receptor antagonist delivered directly and by gene therapy inhibits matrix degradation in the intact degenerate human intervertebral disc: an in situ zymographic and gene therapy study. Arthritis Res Ther 9, R83
15. Goupille, P., Mulleman, D., and Chevalier, X. (2007) Is interleukin-1 a good target for therapeutic intervention in intervertebral disc degeneration: lessons from the osteoarthritic experience. Arthritis Res Ther 9, 110
16. Lee, J. M., Song, J. Y., Baek, M., Jung, H. Y., Kang, H., Han, I. B., Kwon, Y. D., and Shin, D. E. (2011) Interleukin-1beta induces angiogenesis and innervation in human intervertebral disc degeneration. J Orthop Res 29, 265-269
17. Hoyland, J. A., Le Maitre, C., and Freemont, A. J. (2008) Investigation of the role of IL-1 and TNF in matrix degradation in the intervertebral disc. Rheumatology (Oxford) 47, 809-814
18. Bachmeier, B. E., Nerlich, A. G., Weiler, C., Paesold, G., Jochum, M., and Boos, N. (2007) Analysis of tissue distribution of TNF-alpha, TNF-alpha-receptors, and the activating TNF-alpha-converting enzyme suggests activation of the TNF-alpha system in the aging intervertebral disc. Ann N Y Acad Sci 1096, 44-54
19. Weiler, C., Nerlich, A. G., Bachmeier, B. E., and Boos, N. (2005) Expression and distribution of tumor necrosis factor alpha in human lumbar intervertebral discs: a study in surgical specimen and autopsy controls. Spine (Phila Pa 1976) 30, 44-53; discussion 54
20. Hiyama, A., Mochida, J., Omi, H., Serigano, K., and Sakai, D. (2008) Cross talk between Smad transcription factors and TNF-alpha in intervertebral disc degeneration. Biochem Biophys Res Commun 369, 679-685
21. Holm, S., Mackiewicz, Z., Holm, A. K., Konttinen, Y. T., Kouri, V. P., Indahl, A., and Salo, J. (2009) Pro-inflammatory, pleiotropic, and anti-inflammatory TNF-alpha, IL-6, and IL-10 in experimental porcine intervertebral disk degeneration. Vet Pathol 46, 1292-1300
22. Seguin, C. A., Pilliar, R. M., Madri, J. A., and Kandel, R. A. (2008) TNF-alpha induces MMP2 gelatinase activity and MT1-MMP expression in an in vitro model of nucleus pulposus tissue degeneration. Spine (Phila Pa 1976) 33, 356-365
23. Richardson, S. M., Doyle, P., Minogue, B. M., Gnanalingham, K., and Hoyland, J. A. (2009) Increased expression of matrix metalloproteinase-10, nerve growth factor and substance P in the painful degenerate intervertebral disc. Arthritis Res Ther 11, R126
24. Goupille, P., Jayson, M. I., Valat, J. P., and Freemont, A. J. (1998) Matrix metalloproteinases: the clue to intervertebral disc degeneration? Spine (Phila Pa 1976) 23, 1612-1626
25. Kohyama, K., Saura, R., Doita, M., and Mizuno, K. (2000) Intervertebral disc cell apoptosis by nitric oxide: biological understanding of intervertebral disc degeneration. Kobe J Med Sci 46, 283-295
26. Poveda, L., Hottiger, M., Boos, N., and Wuertz, K. (2009) Peroxynitrite induces gene expression in intervertebral disc cells. Spine (Phila Pa 1976) 34, 1127-1133
27. Burke, J. G., RW, G. W., Conhyea, D., McCormack, D., Dowling, F. E., Walsh, M. G., and Fitzpatrick, J. M. (2003) Human nucleus pulposis can respond to a pro-inflammatory stimulus. Spine (Phila Pa 1976) 28, 2685-2693
28. Podichetty, V. K. (2007) The aging spine: the role of inflammatory mediators in intervertebral disc degeneration. Cell Mol Biol (Noisy-le-grand) 53, 4-18
29. Seguin, C. A., Pilliar, R. M., Roughley, P. J., and Kandel, R. A. (2005) Tumor necrosis factor-alpha modulates matrix production and catabolism in nucleus pulposus tissue. Spine (Phila Pa 1976) 30, 1940-1948
30. Horii, M., Orita, S., Nagata, M., Takaso, M., Yamauchi, K., Yamashita, M., Inoue, G., Eguchi, Y., Ochiai, N., Kishida, S., Aoki, Y., Ishikawa, T., Arai, G., Miyagi, M., Kamoda, H., Kuniyoshi, K., Suzuki, M., Nakamura, J., Toyone, T., Takahashi, K., and Ohtori, S. (2011) Direct application of the tumor necrosis factor-alpha inhibitor, etanercept, into a punctured intervertebral disc decreases calcitonin gene-related peptide expression in rat dorsal root ganglion neurons. Spine (Phila Pa 1976) 36, E80-85
31. Kepler, C. K., Markova, D. Z., Dibra, F., Yadla, S., Vaccaro, A. R., Risbud, M. V., Albert, T. J., and Anderson, D. G. (2013) Expression and relationship of proinflammatory chemokine RANTES/CCL5 and cytokine IL-1beta in painful human intervertebral discs. Spine (Phila Pa 1976) 38, 873-880
32. Biyani, A., and Andersson, G. B. (2004) Low back pain: pathophysiology and management. J Am Acad Orthop Surg 12, 106-115
33. Hayashi, S., Taira, A., Inoue, G., Koshi, T., Ito, T., Yamashita, M., Yamauchi, K., Suzuki, M., Takahashi, K., and Ohtori, S. (2008) TNF-alpha in nucleus pulposus induces sensory nerve growth: a study of the mechanism of discogenic low back pain using TNF-alpha-deficient mice. Spine (Phila Pa 1976) 33, 1542-1546
34. Yamashita, M., Ohtori, S., Koshi, T., Inoue, G., Yamauchi, K., Suzuki, M., and Takahashi, K. (2008) Tumor necrosis factor-alpha in the nucleus pulposus mediates radicular pain, but not increase of inflammatory peptide, associated with nerve damage in mice. Spine (Phila Pa 1976) 33, 1836-1842
35. Purmessur, D., Freemont, A. J., and Hoyland, J. A. (2008) Expression and regulation of neurotrophins in the nondegenerate and degenerate human intervertebral disc. Arthritis Res Ther 10, R99
36. Sinclair, S. M., Shamji, M. F., Chen, J., Jing, L., Richardson, W. J., Brown, C. R., Fitch, R. D., and Setton, L. A. (2011) Attenuation of inflammatory events in human intervertebral disc cells with a tumor necrosis factor antagonist. Spine (Phila Pa 1976) 36, 1190-1196
37. Moon, H. J., Kim, J. H., Lee, H. S., Chotai, S., Kang, J. D., Suh, J. K., and Park, Y. K. (2012) Annulus fibrosus cells interact with neuron-like cells to modulate production of growth factors and cytokines in symptomatic disc degeneration. Spine (Phila Pa 1976) 37, 2-9
38. Hamamoto, H., Miyamoto, H., Doita, M., Takada, T., Nishida, K., and Kurosaka, M. (2012) Capability of nondegenerated and degenerated discs in producing inflammatory agents with or without macrophage interaction. Spine (Phila Pa 1976) 37, 161-167
39. Jung, W. W., Kim, H. S., Shon, J. R., Lee, M., Lee, S. H., Sul, D., Na, H. S., Kim, J. H., and Kim, B. J. (2011) Intervertebral disc degeneration-induced expression of pain-related molecules: glial cell-derived neurotropic factor as a key factor. J Neurosurg Anesthesiol 23, 329-334
40. Ohtori, S., Inoue, G., Eguchi, Y., Orita, S., Takaso, M., Ochiai, N., Kishida, S., Kuniyoshi, K., Aoki, Y., Nakamura, J., Ishikawa, T., Arai, G., Miyagi, M., Kamoda, H., Suzuki, M., Sakuma, Y., Oikawa, Y., Kubota, G., Inage, K., Sainoh, T., Toyone, T., Yamauchi, K., Kotani, T., Akazawa, T., Minami, S., and Takahashi, K. (2013) Tumor necrosis factor-alpha-immunoreactive cells in nucleus pulposus in adolescent patients with lumbar disc herniation. Spine (Phila Pa 1976) 38, 459-462
41. Schroeder, M., Viezens, L., Schaefer, C., Friedrichs, B., Algenstaedt, P., Ruther, W., Wiesner, L., and Hansen-Algenstaedt, N. (2013) Chemokine profile of disc degeneration with acute or chronic pain. J Neurosurg Spine 18, 496-503
42. Krock, E., Rosenzweig, D. H., Chabot-Dore, A. J., Jarzem, P., Weber, M. H., Ouellet, J. A., Stone, L. S., and Haglund, L. (2014) Painful, degenerating intervertebral discs up-regulate neurite sprouting and CGRP through nociceptive factors. J Cell Mol Med 18, 1213-1225
43. Ohtori, S., Inoue, G., Miyagi, M., and Takahashi, K. (2015) Pathomechanisms of discogenic low back pain in humans and animal models. Spine J 15, 1347-1355
44. Vadala, G., Russo, F., Musumeci, M., D'Este, M., Cattani, C., Catanzaro, G., Tirindelli, M. C., Lazzari, L., Alini, M., Giordano, R., and Denaro, V. (2016) A clinically relevant hydrogel based on hyaluronic acid and platelet rich plasma as a carrier for mesenchymal stem cells: Rheological and biological characterization. Journal of orthopaedic research : official publication of the Orthopaedic Research Society

## Claims

1. Cannabidiol for use in a method of treating a degenerative disc disease in an individual, said method comprising administering the cannabidiol and at least one therapeutic intervention for a degenerative disc disease to an individual at the same time, and said therapeutic intervention comprising a therapeutically effective amount of fibroblast cells.

2. Cannabidiol for use according to claim 1, wherein the fibroblast cells are from one or more tissues selected from the group consisting of adipose tissue, dermal tissue, bone marrow, peripheral blood, Wharton's Jelly, placenta, omentum, mobilized peripheral blood, and a combination thereof, and/or wherein the fibroblasts cells are from dermal tissue.

3. Cannabidiol for use according to claim 1 or claim 2, wherein the cannabidiol is administered systemically, or locally into a disc of the individual.

4. Cannabidiol for use according to any one of claims 1 to 3, wherein the cannabidiol is administered at a dosage and frequency sufficient to reduce apoptosis of cells in the nucleus pulposus.

5. Cannabidiol for use according to claim 4, wherein the cells in the nucleus pulposus are selected from the group consisting of chondrocytes, notochord cells, notochord progenitor cells, chondrocyte progenitor cells, and a combination thereof.

6. Cannabidiol for use according to claim 4, wherein the cells in the nucleus pulposus comprise exogenously administered cells.

7. Cannabidiol for use according to claim 4, wherein the cells in the nucleus pulposus comprise the therapeutic intervention for a degenerative disc disease.

8. Cannabidiol for use according to claim 6, wherein said exogenously administered cells are fibroblast regenerative cells.

9. Cannabidiol for use according to any one of claims 1-8, wherein:
(a) the fibroblasts are proliferating at a rate of 14-21 hours per cell multiplication; and/or
(b) the fibroblasts secrete 0.1 pg-77 pg of interleukin 1 per culture of 1 million fibroblasts at 75% confluence on a surface, or 1 pg-500 pg of interleukin FGF-1 per culture of 1 million fibroblasts at 75% confluence on a surface; and/or
(c) the fibroblasts decrease ability of responding T cells to proliferate in a mixed lymphocyte reaction, optionally wherein said decrease in proliferation comprises a decrease of more than 20% as compared to a control mixed lymphocyte reaction in which fibroblasts are not added; and/or
(d) the fibroblasts are treated with human chorionic gonadatropin (hCG) to augment immune modulatory activity, optionally wherein said hCG is administered to cells at a concentration of 1 nM to 1 µM, or of 10 nM to 100 nM; and/or
(e) said fibroblasts are treated with oxytocin to augment immune modulatory activity, optionally wherein said oxytocin is administered to cells at a concentration of 1 nM to 10 µM, or100 nM to 1 µM.

10. Cannabidiol for use according to any one of claims 1-9, wherein:
(a) the cannabidiol comprises (-)-cannabidiol, or the cannabidiol consists of (-)-cannabidiol; and/or
(b) the effective amount of cannabidiol is between about 50 mg and about 500 mg total daily; and/or
(c) the cannabidiol comprises a gel formulation, optionally wherein the gel formulation comprises a permeation-enhanced gel; and/or
(d) administering the cannabidiol and/or therapeutic intervention comprises administering a single daily dose to the individual, or two daily doses to the individual; and/or
(e) administering the cannabidiol and/or therapeutic intervention comprises transdermally administering the cannabidiol to the individual; and/or
(f) administering the cannabidiol comprises intradiscally administering the cannabidiol to the individual.

11. Cannabidiol for use according to any one of claims 1-10, wherein said cannabidiol is administered together with a localization composition comprising one or more extracellular matrix molecules.

12. Cannabidiol for use according to claim 11, wherein the one or more extracellular matrix molecules provides sustained release.

13. Cannabidiol for use according to claim 12, wherein said extracellular matrix is hyaluronic acid, optionally wherein said hyaluronic acid is comprised of long chained hyaluronic acid molecules.

## Patentansprüche

1. Cannabidiol für die Verwendung in einem Verfahren zum Behandeln einer degenerativen Bandscheibenerkrankung bei einem Individuum, wobei das Verfahren das gleichzeitige Verabreichen des Cannabidiols und wenigstens einer therapeutischen Intervention für eine degenerative Bandscheibenerkrankung an ein Individuum umfasst und die therapeutische Intervention eine therapeutisch wirksame Menge an Fibroblastenzellen umfasst.

2. Cannabidiol für die Verwendung nach Anspruch 1, wobei die Fibroblasten aus einem oder mehreren Geweben stammen, die aus der Gruppe ausgewählt sind, die aus Fettgewebe, Hautgewebe, Knochenmark, peripherem Blut, Wharton-Gel, Plazenta, Omentum, mobilisiertem peripherem Blut und einer Kombination davon besteht, und/oder wobei die Fibroblasten aus Hautgewebe stammen.

3. Cannabidiol für die Verwendung nach Anspruch 1 oder 2, wobei das Cannabidiol systemisch oder lokal in eine Bandscheibe des Individuums verabreicht wird.

4. Cannabidiol für die Verwendung nach einem der Ansprüche 1 bis 3, wobei das Cannabidiol in einer Dosierung und Häufigkeit verabreicht wird, die ausreichen, um die Apoptose von Zellen in dem Nucleus pulposus zu reduzieren.

5. Cannabidiol für die Verwendung nach Anspruch 4, wobei die Zellen in dem Nucleus pulposus aus der Gruppe ausgewählt sind, die aus Chondrozyten, Notochordzellen, Notochord-Vorläuferzellen, Chondrozyten-Vorläuferzellen und einer Kombination davon besteht.

6. Cannabidiol für die Verwendung nach Anspruch 4, wobei die Zellen in dem Nucleus pulposus exogen verabreichte Zellen umfassen.

7. Cannabidiol für die Verwendung nach Anspruch 4, wobei die Zellen in dem Nucleus pulposus die therapeutische Intervention für eine degenerative Bandscheibenerkrankung umfassen.

8. Cannabidiol für die Verwendung nach Anspruch 6, wobei die exogen verabreichten Zellen Fibroblasten-Regenerationszellen sind.

9. Cannabidiol für die Verwendung nach einem der Ansprüche 1-8, wobei:
(a) die Fibroblasten sich mit einer Rate von 14 bis 21 Stunden pro Zellvermehrung vermehren; und/oder
(b) die Fibroblasten 0,1 pg-77 pg Interleukin 1 pro Kultur von 1 Million Fibroblasten bei einer Konfluenz von 75 % auf einer Oberfläche oder 1 pg-500 pg Interleukin FGF-1 pro Kultur von 1 Million Fibroblasten bei einer Konfluenz von 75 % auf einer Oberfläche sekretieren; und/oder
(c) die Fibroblasten die Fähigkeit der reagierenden T-Zellen verringern, sich in einer gemischten Lymphozytenreaktion zu vermehren, wobei die Verringerung der Vermehrung optional eine Verringerung von mehr als 20 % im Vergleich zu einer gemischten Lymphozytenreaktion umfasst, in der keine Fibroblasten hinzugefügt wurden; und/oder
(d) die Fibroblasten mit humanem Choriongonadotropin (hCG) behandelt werden, um die immunmodulatorische Aktivität zu verstärken, wobei optional das hCG den Zellen in einer Konzentration von 1 nM bis 1 µM oder von 10 nM bis 100 nM verabreicht wird; und/oder
(e) die Fibroblasten mit Oxytocin behandelt werden, um die immunmodulatorische Aktivität zu verstärken, wobei optional das Oxytocin den Zellen in einer Konzentration von 1 nM bis 10 µM oder von 100 nM bis 1 µM verabreicht wird.

10. Cannabidiol für die Verwendung nach einem der Ansprüche 1-9, wobei:
(a) das Cannabidiol (-)-Cannabidiol umfasst oder das Cannabidiol aus (-)-Cannabidiol besteht; und/oder
(b) die wirksame Menge an Cannabidiol zwischen etwa 50 mg und etwa 500 mg täglich beträgt; und/oder
(c) das Cannabidiol eine Gelformulierung umfasst, wobei die Gelformulierung optional ein permeationsverstärktes Gel umfasst; und/oder
(d) das Verabreichen des Cannabidiols und/oder die therapeutische Intervention das Verabreichen einer einzigen täglichen Dosis an das Individuum oder zwei täglicher Dosen an das Individuum umfasst; und/oder
(e) das Verabreichen des Cannabidiols und/oder die therapeutische Intervention das transdermale Verabreichen des Cannabidiols an das Individuum umfasst; und/oder
(f) das Verabreichen des Cannabidiols das intradiskale Verabreichen des Cannabidiols an das Individuum umfasst.

11. Cannabidiol für die Verwendung nach einem der Ansprüche 1-10, wobei das Cannabidiol zusammen mit einer Lokalisierungszusammensetzung verabreicht wird, die ein oder mehrere extrazelluläre Matrixmoleküle umfasst.

12. Cannabidiol für die Verwendung nach Anspruch 11, wobei das eine oder die mehreren extrazellulären Matrixmoleküle eine verzögerte Freisetzung bereitstellen.

13. Cannabidiol für die Verwendung nach Anspruch 12, wobei die extrazelluläre Matrix Hyaluronsäure ist, wobei die Hyaluronsäure optional aus langkettigen Hyaluronsäuremolekülen besteht.

## Revendications

1. Cannabidiol destiné à être utilisé dans une méthode de traitement d'une maladie discale dégénérative chez un individu, ladite méthode comprenant l'administration simultanée du cannabidiol et d'au moins une intervention thérapeutique pour une maladie discale dégénérative à un individu, et ladite intervention thérapeutique comprenant une quantité thérapeutiquement efficace de cellules fibroblastiques.

2. Cannabidiol destiné à être utilisé selon la revendication 1, dans lequel les cellules fibroblastiques proviennent d'un ou plusieurs tissus choisis dans le groupe constitué par : tissu adipeux, tissu cutané, moelle osseuse, sang périphérique, gelée de Wharton, placenta, épiploon, sang périphérique mobilisé et une combinaison de ceux-ci, et/ou les cellules fibroblastiques proviennent de tissu cutané.

3. Cannabidiol destiné à être utilisé selon la revendication 1 ou la revendication 2, dans lequel le cannabidiol est administré par voie systémique ou localement dans un disque de l'individu.

4. Cannabidiol destiné à être utilisé selon l'une quelconque des revendications 1 à 3, dans lequel le cannabidiol est administré à une dose et à une fréquence suffisantes pour réduire l'apoptose des cellules du noyau pulpeux.

5. Cannabidiol destiné à être utilisé selon la revendication 4, dans lequel les cellules du noyau pulpeux sont choisies dans le groupe constitué par : chondrocytes, cellules de la notochorde, cellules progénitrices de la notochorde, cellules progénitrices de chondrocytes et une combinaison de ceux-ci.

6. Cannabidiol destiné à être utilisé selon la revendication 4, dans lequel les cellules du noyau pulpeux comprennent des cellules administrées de manière exogène.

7. Cannabidiol destiné à être utilisé selon la revendication 4, dans lequel les cellules du noyau pulpeux comprennent l'intervention thérapeutique pour une maladie discale dégénérative.

8. Cannabidiol destiné à être utilisé selon la revendication 6, dans lequel lesdites cellules administrées de manière exogène sont des cellules fibroblastiques régénératives.

9. Cannabidiol destiné à être utilisé selon l'une quelconque des revendications 1 et 8, dans lequel
(a) les fibroblastes prolifèrent à un taux de 14 à 21 heures par multiplication cellulaire, et/ou
(b) les fibroblastes sécrètent 0,1 à 77 pg d'interleukine 1 par culture d'un million de fibroblastes à 75 % de confluence sur une surface, ou 1 à 500 pg d'interleukine FGF-1 par culture d'un million de fibroblastes à 75 % de confluence sur une surface, et/ou
(c) les fibroblastes diminuent la capacité des lymphocytes T réactifs à proliférer dans une réaction lymphocytaire mixte, ladite diminution de la prolifération comprenant éventuellement une diminution de plus de 20 % par rapport à une réaction lymphocytaire mixte témoin dans laquelle aucun fibroblaste n'est ajouté, et/ou
(d) les fibroblastes sont traités avec de la gonadotrophine chorionique humaine (hCG) pour augmenter l'activité immunomodulatrice, ladite hCG étant éventuellement administrée aux cellules à une concentration de 1 nM à 1 µM ou de 10 nM à 100 nM, et/ou
(e) lesdits fibroblastes sont traités avec de l'oxytocine pour augmenter l'activité immunomodulatrice, ladite oxytocine étant éventuellement administrée aux cellules à une concentration de 1 nM à 10 µM ou de 100 nM à 1 µM.

10. Cannabidiol destiné à être utilisé selon l'une quelconque des revendications 1 à 9, dans lequel
(a) le cannabidiol comprend du (-)-cannabidiol ou le cannabidiol est constitué de (-)-cannabidiol, et/ou
(b) la quantité efficace de cannabidiol se situe entre environ 50 mg et environ 500 mg par jour au total, et/ou
(c) le cannabidiol comprend une formulation de gel, la formulation de gel comprenant éventuellement un gel à perméation améliorée, et/ou
(d) l'administration du cannabidiol et/ou de l'intervention thérapeutique comprend l'administration d'une seule dose quotidienne à l'individu ou de deux doses quotidiennes à l'individu, et/ou
(e) l'administration du cannabidiol et/ou de l'intervention thérapeutique comprend l'administration transdermique du cannabidiol à l'individu, et/ou
(f) l'administration du cannabidiol comprend l'administration intradiscale du cannabidiol à l'individu.

11. Cannabidiol destiné à être utilisé selon l'une quelconque des revendications 1 à 10, ledit cannabidiol étant administré conjointement avec une composition de localisation comprenant une ou plusieurs molécules de matrice extracellulaire.

12. Cannabidiol destiné à être utilisé selon la revendication 11, dans lequel la ou les molécules de matrice extracellulaire assurent une libération prolongée.

13. Cannabidiol destiné à être utilisé selon la revendication 12, dans lequel ladite matrice extracellulaire est de l'acide hyaluronique, ledit acide hyaluronique étant éventuellement composé de molécules d'acide hyaluronique à longue chaîne.
